Europäisches Patentamt

European Patent Office

Office européen des brevets

(11) Publication number: **0 082 880**
**B1**

# EUROPEAN PATENT SPECIFICATION

(45) Date of publication of patent specification: **11.06.86**

(51) Int. Cl.⁴: **A 61 L 15/03**

(21) Application number: **82902505.5**

(22) Date of filing: **08.07.82**

(86) International application number:
**PCT/US82/00926**

(87) International publication number:
**WO 83/00092 20.01.83 Gazette 83/02**

(54) **POLYMERIC DIFFUSION MATRIX CONTAINING PROPRANOLOL.**

(30) Priority: **08.07.81 US 281322**
**06.01.82 US 337386**
**11.01.82 US 338256**

(43) Date of publication of application:
**06.07.83 Bulletin 83/27**

(45) Publication of the grant of the patent:
**11.06.86 Bulletin 86/24**

(84) Designated Contracting States:
**AT BE CH DE FR GB LI LU NL SE**

(56) References cited:
**FR-M- 6 733**
**GB-A- 493 561**
**US-A-2 155 658**
**US-A-2 160 503**
**US-A-2 693 438**
**US-A-3 287 222**
**US-A-3 742 951**
**US-A-3 972 995**
**US-A-4 210 633**
**US-A-4 291 015**

(73) Proprietor: **Key Pharmaceuticals, Inc.**
**18425 N.W. 2nd Avenue.**
**Miami, Florida 33169 (US)**

(72) Inventor: **KEITH, Alec Dell**
**18425 N.W. 2nd Avenue**
**Miami, FL 33169 (US)**
Inventor: **SNIPES, Wallace**
**P.O. Box 298**
**Pine Grove Mills, PA 16801 (US)**

(74) Representative: **Werner, Hans-Karsten, Dr. et al**
**Deichmannhaus am Hauptbahnhof**
**D-5000 Köln 1 (DE)**

(56) References cited:
**Chemical Abstracts Volume 94, Issued 1981,
(Columbus, Ohio, USA), See page 311, column
2, the Abstract 94: 20412E, KEITH et al, (KEY
PHARMACEUTICALS, INC.) EUR. PAT. APPL.
13,606, 23 July 1980, "POLYMERIC DIFFUSION
MATRIX AND DRUG DELIVERY DEVICE
COMPRISING SAID MATRIX"**

Courier Press, Leamington Spa, England.

**0 082 880**

(56) References cited:

Chemical Abstracts, Volume 92, Issued 1980, (Columbus, Ohio, USA), See page 350, column 2, Abstract 92: 169275D, ANIKAWA et al, JAPAN KOKAI TOKYO KOHO 79,151,115, 28 Nov. 1979, "MEDICATED WET PAKCS"

Chemical Abstracts, Volume 89, Issued 1978, (Columbus, Ohio, USA), See page 548, column 1, Abstract 89: 117911b, ARAI et al, JAPAN KOKAI 78,50,320, 08 May 1978, "HYDROPHILIC PLASTERS"

Chemical Abstracts, Volume 87, Issued 1977, (Columbus, Ohio, USA), See page 330, column 1, Abstract 87: 141303J, TAURA et al, JAPAN KOKAI 77, 38,016, 24 March 1977, "POULTICES"

Chemical Abstracts, Volume 86, Issued 1977, (Columbus, Ohio, USA), See page 380, columns 1 and 2, Abstract 86: 161344F SASUKI et al, JAPAN KOKAI 76,112,511, 26 March 1975, "CATAPLASM"

Chemical Abstracts, Volume 47, Issued 1953, (Columbus, Ohio, USA), See column 7165 DEF, ITO et al, BULL. PHARM. RESEARCH INST. JAPAN No. 2: 1.12, "PHARMACEUTICAL STUDIES ON OINTMENTS AND EXTERNAL REMEDIES"

## Description

Summary of the Invention

The present invention relates to a polymeric diffusion matrix containing 1-isopropylamino-3-(1-naphthyloxy)-2-propanol, also known as "propranolol". More particularly, the invention relates to a polymeric diffusion matrix containing propranolol characterized by a sustained release of the propranolol. Propranolol is a widely used beta-adrenergic blocking agent, indicated for the control of hypertension, arrhythmias, and migraine. By the term "propranolol" there is contemplated a mixture of the D and L forms, although the L form is recognized as the preferred therapeutic agent. While the L form may be used alone, for a prolonged sustained release product it is also contemplated that a mixture of the D and L forms could be used.

A self-supporting polymeric diffusion matrix is provided for the sustained release of propranolol in order to deliver said propranolol to a patient said matrix comprising from 1 to 60% by weight of a polar plasticizer, preferably glycerol and/or polyethyleneglycol, 2 to 30% by weight polyvinylpyrrolidone, and a pharmaceutically effective amount of the propranolol to provide a sustained release of said propranolol over a prolonged period characterized in that said matrix further comprises from 5 to 20% by weight polyvinylalcohol having a molecular weight from 50,000 to 150,000, from 10 to 25% by weight polyvinylalcohol having a molecular weight from 4,000 to 15,000, and from 5 to 20% by weight solubilizing agent.

Polar plasticizers suitable for use in this invention include principally poly-loweralkylene oxides, but other polar plasticizers such as diethylphthalic diethylphthalate may be used.

In one embodiment the polar plasticizer is glycerol present in an amount of from 2 to 60% by weight. In another embodiment the polar plasticizer is polyethyleneglycol present in an amount of from 1 to 15% by weight. A still further embodiment contemplates a mixture of glycerol and polyethyleneglycol wherein the latter is present in an amount by weight of from 1 to 5 parts per weight glycerol.

The self-supporting polymeric diffusion matrix contains a mixture of polyvinylalcohol and polyvinylpyrrolidone. However, the composition may also contain from 1 to 9% agar or agarose, and preferably from 1.5 to 3% agar or agarose, 2% agar or agarose being particularly preferred.

As the higher molecular weight, polyvinylalcohol used in the present invention, there is contemplated one having a molecular weight from 50,000 to 150,000, and more preferably 100,000 to 150,000, 115,000 having been used in related systems of the present inventors with success. The polyvinylalcohol should be hydrolyzed, generally at least to the extent of 90% with a preferred embodiment being at least 95% hydrolyzed.

The higher molecular weight polyvinylalcohol component is present in the final composition in an amount of from 5 to 20% by weight, and preferably about 10% by weight. The lower molecular weight component is present in an amount of from 10 to 25% by weight, and preferably about 15% by weight. The molecular weight ranges from 4,000 to 15,000, with 10,000 being a preferred embodiment. The degree of hydrolysis of this lower molecular weight portion is preferably at least about 75%, and preferably about 88%. Optionally included is a solubilizing agent which functions to bring the components into solution, which is present preferably in an amount of from 5 to 20% by weight, diethanolmyristoylamide being a preferred embodiment.

The polyvinylpyrrolidone should have a molecular weight of from 15,000 to 85,000, and more preferably from 20,000 to 60,000. Polyvinylpyrrolidone with a molecular weight of 40,000 is a particularly preferred embodiment.

The amount by weight of the ingredients other than the polar plasticizer generally should be in the following ranges: Polyvinylalcohol is generally present in a amount up to 30% by weight, with 20% being a preferred embodiment; polyvinylpyrrolidone is present generally in an amount of from 2 to 30% by weight, with about 10% being preferred.

In particular embodiments of this invention the total amount of polyvinylalcohol and polyvinylpyrrolidone used is from 25% to 50% by weight.

Polyalkylene glycols (poly-loweralkylene oxides) such as polyethyleneglycol and polypropylene glycol may replace all or part of the glycerol.

In forming the matrix, excess water is not required. In accordance with a preferred aspect of the present invention, about 2% by weight propranolol is included in the diffusion matrix. The resultant homogeneous mixture is poured into forms preferably made of glass or stainless steel. For transdermal application a diffusion matrix with a thickness of 1 to 3 mm is in accordance with a preferred aspect of this invention. This diffusion matrix can be cut to obtain the desired surface area once it is suitably cured.

The following methods may be used for preparing the diffusion matrix of the present invention.

In one method, the matrix is formed at atmospheric pressure. Water and polar plasticizer are first mixed together. A polar plasticizer such as glycerol or polyethyleneglycol component is used in the matrix. A matrix formed without a polar plasticizer is not flexible and has poor diffusional contact with the skin, causing unreliable diffusion release. The polyvinylalcohol and polyvinylpyrrolidone are then added to the polar plasticizer water mixture at room temperature with agitation. The mixture is heated to a temperature within the range of from 90 to 95°C at atmospheric pressure to extend the polymers. If desired, the mixture may be maintained at an elevated temperature for a period of time, based on polymer stability, prior to addition of the drug. Thus, the mixture is stable for a period of time and may be kept for such a period before being mixed with the drug to be delivered to the patient. Thereafter, the mixture is temperature-adjusted and the drug to be applied to the patient is then added to the mixture, with thorough agitation. Once a homogeneous mixture of the polymer solution and drug is obtained, the mixture is ready to be cast to form in a drug-containing diffusion matrix. After casting, the mixture is cooled to a temperature such that gelation occurs.

In another method, the polymeric material is heated under pressure to accomplish dissolution in the mixture, the propranolol is mixed in and the material is extruded under pressure into a mold of suitable size and geometry. The use of pressure allows for the incorporation of higher amounts of polymeric material into the matrix, up to 60% total polyvinylpyrrolidone and polyvinylalcohol content, thus improving film strength content, and dimensional stability and allowing for thinner matrices. This pressure method further reduces or eliminates altogether curing and/or drying time.

In a further embodiment there is provided a pH buffer having a sufficiently large molecular structure so that it would not pass through the skin, thus providing a sufficiently high pH to operate in the preferred aspect of the invention, without having base molecules pass through the skin. A pH of at least 7.5 is preferred. This aspect of the invention is accomplished through the provision of Eudragit® polymers, charged polymers such as poly(methyl methacrylate) and poly(acrylic acid). In one embodiment polymethacrylic acid saturated with choline is used as the polymer, e.g., in an amount of 10% by weight of the total diffusion matrix composition. Thus, for example, such a polymer may be added to the ingredients of Example I below to provide a pH stabilized diffusion matrix to facilitate the diffusion of the propranolol through the skin by maintaining a desirably high enough pH.

It has been further found that curing is facilitated by subjecting the matrix to a temperature down to about −20°C immediately after casting, especially when polyethyleneglycol is used as the plasticizer. The setting time is quickened considerably.

Sodium dodecyl sulfate or sorbitan (Tween-20)® or other detergents may be added in an amount of 0.1 to 10% by weight, based on the matrix, as a dispersing agent, if desired. Up to 10% of one or more absorption facilitators to insure skin penetration such as dimethylsulfoxide, decylmethylsulffoxime, or other penetration enhancers may also be added. Suitable preservatives, such as sodium benzoate, may be also added where indicated.

The present drug delivery device comprises the drug-containing diffusion matrix which can be applied as a transdermal patch with means for fastening the matrix to the skin of a patient. Such means can take various forms, such as an occlusive backing layer forming a kind of "bandage" with the diffusion matrix being held against the skin of a patient being treated. A polyethylene or Mylar® tape is contemplated as one form of occlusive layer in accordance with the present invention. It can also take the form of an elastic band, such as a cloth band, a rubbery band, or other material. Here, the diffusion matrix is placed directly on the skin and held in place over the arm or wrist of the patient. An intermediate adhesive layer between the diffusion matrix and the skin capable of permitting the transdermal application of the drug can also be used.

The invention is illustrated by the following non-limiting example:

Example I

Together there are mixed 20 g glycerol and 55 ml water. This mixture is heated to 90°C; after reaching at least 70°C, there are slowly added 8 g polyvinylalcohol (PVA 100% hydrolyzed, molecular weight 115,000) and 7 g polyvinylalcohol having a molecular weight of 10,000 (88% hydrolyzed) and 8 g polyvinylpyrrolidone (mw 40,000). In addition 10% by weight of diethanolmyristoylamide are included. The mixture is stirred at 90°C until solution is effected, which may take about 10 minutes; it will be appreciated that with large quantities, a considerably longer period of time may be needed. 98 ml of this solution is then mixed with 2 g propranolol, this mixture then being mechanically stirred until homogeneous. The homogeneous mixture is then poured into forms made of glass or stainless steel which serve as templates to produce a diffusion matrix having a thickness of 0.2 to 2 mm. This diffusion matrix is then cut into square pieces of about 2,54 cm on each side, i.e., to provide a total surface area of about 6.5 cm$^2$.

The diffusion matrix is applied to the skin of a patient in need of a beta-blocking effect, the propranolol being transdermally delivered. The diffusion matrix is ideally applied to the skin of the patient by means of a single-piece bandage having the diffusion matrix in the center under the occlusive layer, the bandage being provided to the patient with a peel-off cover much like a "band-aid".

**Claims**

1. A self-supporting polymeric diffusion matrix for the sustained release of propranolol in order to deliver said propranolol to a patient, said matrix comprising from 1 to 60% by weight of a polar plasticizer, preferably glycerol and/or polyethyleneglycol, from 2 to 30% by weight polyvinylpyrrolidone, and a pharmaceutically effective amount of the propranolol to provide a sustained release of said propranolol over a prolonged period characterized in that said matrix further comprises from 5 to 20% by weight polyvinylalcohol having a molecular weight from 50,000 to 150,000, from 10 to 25% by weight polyvinylalcohol having a molecular weight from 4,000 to 15,000, and from 5 to 20% by weight solubilizing agent.

2. The self-supporting polymeric diffusion matrix of claim 1, characterized in that said solubilizing agent is diethanolmyristoylamide.

**Revendications**

1. Une matrice de diffusion polymérique autoporteuse pour la libération progressive de propranolol afin d'administrer ledit propranolol à un patient, ladite matrice comprenant de 1 à 60% en poids d'un plastifiant polaire, de préférence le glycérol et/ou le polyéthylèneglycol, de 2 à 30% en poids de polyvinylpyrrolidone, et une quantité efficace pharmaceutiquement de propranolol pour obtenir une libération progressive dudit propranolol sur une période de temps prolongée, caractérisée par le fait que ladite matrice comprend en outre de 5 à 20% en poids d'alcool polyvinylique ayant un poids moléculaire de 50.000 à 150.000, de 10 à 25% en poids d'alcool polyvinylique ayant un poids moléculaire de 4.000 à 15.000 et de 5 à 20% en poids d'un agent de solubilisation.

2. La matrice de diffusion polymérique autoporteuse de la revendication 1, caractérisée en ce que ledit agent de solubilisation est le diéthanolmyristoylamide.

**Patentansprüche**

1. Selbsttragende polymere Diffusionsmatrix für die Langzeit-Abgabe von Propranolol zur Verabreichung des Propanolols an einen Patienten, wobei die Matrix 1 bis 60 Gew.-% eines polaren Weichmachers, vorzugsweise Glycerin und/oder Polyethylenglycol, 2 bis 30 Gew-.% Polyvinylpyrrolidon sowie eine pharmazeutisch wirksame Menge des Propranolols enthält, um eine Dauerabgabe des Propranolols über einen längeren Zeitraum hinweg zu bewirken, dadurch gekennzeichnet, daß die Matrix weiterhin 5 bis 20 Gew.% Polyvinylalkohol mit einem Molekulargewicht von 50,000 bis 150,000, 10 bis 25 Gew.-% Polyvinylalkohol mit einem Molekulargewicht von 4,000 bis 15,000 und 5 bis 20 Gew.-% eines löslichmachenden Mittels enthält.

2. Selbsttragende polymere Diffusionsmatrix nach Anspruch 1, dadurch gekennzeichnet, daß das löslichmachende Mittel Diethanolmyristoylamid ist.